# EUROPEAN PATENT APPLICATION

(11) **EP 0 841 393 A1**
(43) Date of publication of application: **13.05.1998**
(21) Application number: 96922267.8
(22) Date of filing: 09.07.1996
(51) Int. Cl.: C12N 15/12, C12N 15/62, C12N 15/63, C12P 21/02

(54) **HUMAN GALECTIN-4-LIKE PROTEIN AND cDNA ENCODING THE SAME**

(30) Priority: 11.07.1995 JP 174778/95
(71) Applicant: SAGAMI CHEMICAL RESEARCH CENTER, Sagamihara-shi, Kanagawa 229 (JP)
(72) Inventor: KATO, Seishi, Sagamihara-shi, Kanagawa 229 (JP); YAMAGUCHI, Tomoko, Kanagawa 125 (JP); SEKINE, Shingo, Sagamihara-shi, Kanagawa 229 (JP); KAMATA, Kouju, Sagamihara-shi, Kanagawa 228 (JP)
(74) Representative: Dost, Wolfgang, Dr.rer.nat., Dipl.-Chem.
(86) International application number: JP9601899
(87) International publication number: WO9703190

(57) **Abstract**

A galectin-4 (a lactose-binding protein)-like protein expressed specifically in the stomach and intestines and a human cDNA encoding the same. The protein is one containing the amino acid sequence represented by SEQ ID NO: 1 while the gene is a cDNA containing the base sequence represented by SEQ ID NO: 2. The protein which is the expression product of this cDNA has a lactose-binding activity and is applicable to drugs and research reagents.

## Description

### Application Field

The present invention relates to a novel cDNA originating from an mRNA expressed in human cells and a galectin-4-like protein encoded by this cDNA. The human cDNA of the present invention can be used as a probe for the gene diagnosis and a gene source for the gene therapy. Furthermore, the cDNA can be used as a gene source for large-scale production of the protein encoded by said cDNA. The protein of the present invention can be used as pharmaceuticals or reagents for glycogenic researches.

### Prior Art

Galectins are the general term for animal lectins binding to galactose. Animal lectins exist in many sites such as the cytoplasm, the nucleus, the cell membrane surface, etc., and considered to be related with the cell proliferation, the differentiation, the canceration, the metastasis, the immunity, and so on [Drickamer, K., Annu. Rev. Cell. Biol., 9: 237-264 (1993)]. Among them, galectin-4 has been found as a lectin contained abundantly in the rat intestinal extract. The galectin-4 is expressed specifically in the digestive tracts such as the stomach and intestines, and is abundant in the mucous membrane, thereby being a putative protein essential for maintaining the functions of these organs. Although a rat cDNA encoding the galectin-4 has been cloned up to date [Oda, Y. et al., J. Biol. Chem., 268: 5929-5939 (1993)], any report has not been presented on a cDNA encoding a human galectin-4-like protein.

### Disclosure of the Invention

As the result of intensive studies, the present inventors were successful in cloning of a human cDNA encoding a galectin-4-like protein, thereby completing the present invention. That is to say, the present invention provides a protein containing the amino acid sequence represented by Sequence No. 1 that is a human galectin-4-like protein. The present invention, also, provides a DNA encoding said protein exemplified as a cDNA containing the base sequence represented by Sequence No. 2 or No. 3.

The human cDNA of the present invention can be cloned from a cDNA library of the human cell origin. This cDNA library is constructed using as a template a poly(A)⁺ RNA extracted from human cells. The human cells may be cells delivered from the human body, for example, by the operation or may be the culture cells. A poly(A)⁺ RNA isolated from the stomach cancer tissue is used in Examples. The cDNA can be synthesized by using any method selected from the Okayama-Berg method [Okayama, H. and Berg, P., Mol. Cell. Biol., 2: 161-170 (1982)], the Gubler-Hoffman method [Gubler, U. and Hoffman, J. Gene, 25: 263-269 (1983)], and so on, but it is preferred to use the capping method [Kato, S. et al., Gene, 150: 243-250 (1994)] as illustrated in Examples in order to obtain a full-length clone in an effective manner. The identification of the cDNA is performed by the determination of the whole base sequence by the sequencing, the search of the amino acid sequence predicted from the base sequence and the known protein having a similar sequence, the protein expression by the in vitro translation, the expression by *Escherichia coli*, and the activity measurement of the expressed product. The activity measurement is carried out by identifying the binding with lactose.

The cDNA of the present invention is characterized by containing the base sequence represented by Sequence No. 1, as exemplified by that represented by Sequence No. 2. For example, that represented by Sequence No. 3 possesses a 1113-bp base sequence with a 972-bp open reading frame. This open reading frame codes for a protein consisting of 323 amino acid residues. This protein possesses such a high 76.3% similarity to the rat galectin-4 in the amino acid sequence level.

Hereupon, the same clone as the cDNA of the present invention can be easily obtained by screening of the human cDNA library constructed from the gastrointestinal tissues or the gastrointestinal cell lines, by the use of an oligonucleotide probe synthesized on the basis of the cDNA base sequence depicted in Sequence No. 3.

In general, the polymorphism due to the individual difference is frequently observed in human genes. Therefore, any cDNA that is subjected to insertion or deletion of one or plural nucleotides and/or substitution with other nucleotides in the base sequence encoding the amino acid sequence represented by Sequence No. 1 or in Sequence No. 3 shall come within the scope of the present invention.

In a similar manner, any protein that is produced by these modifications comprising insertion or deletion of one or plural nucleotides and/or substitution with other nucleotides shall come within the scope of the present invention.

The cDNA of the present invention includes cDNA fragments (more than 10 bp) containing any partial base sequence of the base sequence represented by Sequence No. 2 or No. 3. Also, DNA fragments consisting of a sense chain and an anti-sense chain shall come within this scope. These DNA fragments can be used as the probes for the gene diagnosis.

The protein of the present invention can be expressed in vitro by preparation of an RNA by the in vitro transcription from a vector having a cDNA of the present invention, followed by the in vitro translation using this RNA as a template. Also, the recombination of the translation domain to a suitable expression vector by the method known in the art leads to the expression of a large amount of the encoded protein by using *Escherichia coli*, *Bacillus subtilis*, yeasts, animal cells, and so on. Alternatively, the peptide can be prepared by the chemical synthesis on the basis of the amino acid sequence shown in the sequence table shown below.

Any fusion protein with another optional protein is included in the scope of the present invention, as far as it possesses lactose-binding activity. Such examples include a fusion protein with the maltose-binding protein illustrated in Examples.

### Brief Description of the Drawings

Figure 1 is a figure depicting the structure of the plasmid pHP01049 of the present invention.

Figure 2 is a figure depicting the structure of the *Escherichia coli* expression vector pMKGAL4 of the present invention.

### Best Mode for Carrying Out the Invention

The present invention is embodied in more detail by the following examples, but this embodiment is not intended to restrict the present invention. The basic operations and the enzyme reactions with regard to the DNA recombination are carried out according to the literature [Molecular Cloning. A Laboratory Manual 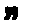 , Cold Spring Harbor Laboratory, 1989]. Unless otherwise stated, restrictive enzymes and a variety of modification enzymes to be used were those available from TAKARA SHUZO. The manufacturer 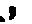 s instructions were used for the buffer compositions as well as for the reaction conditions, in each of the enzyme reactions. The cDNA synthesis was carried out according to the literature [Kato, S. et al., Gene, 150: 243-250 (1994)].

### Examples

### Preparation of Poly(A)⁺ RNA

After 1 g of a human stomach cancer tissue was homogenized in 20 ml of a 5.5 M guanidinium thiocyanate solution, 750 µg of mRNA was prepared according to the literature [Okayama, H. et al., "Methods in Enzymology" Vol. 164, Academic Press, 1987]. This was subjected to oligo(dT)-cellulose column chromatography washed with a 20 mM Tris-hydrochloric acid buffer solution (pH 7.6), 0.5 M NaCl, and 1 mM EDTA to obtain 10 µg of a poly(A)⁺ RNA according to the literature mentioned above.

### Construction of cDNA Library

Ten micrograms of the above described poly(A)⁺ RNA were dissolved in a 100 mM Tris-hydrochloric acid buffer solution (pH 8), one unit of an RNase-free, bacterial alkaline phosphatase was added, and the reaction was run at 37°C for one hour. After the reaction mixture was subjected to phenol extraction followed by ethanol precipitation, the pellet was dissolved in a solution containing 50 mM sodium acetate (pH 6), 1 mM EDTA, 0.1% 2-mercaptoethanol, and 0.01% Triton X-100. Thereto was added one unit of a tobacco acid pyrophosphatase (Epicentre Technologies) and a total 100 µl volume of the resulting mixture was reacted at 37°C for one hour. After the reaction mixture was subjected to phenol extraction followed by ethanol precipitation, the pellet was dissolved in water to obtain a solution of a decapped poly(A)⁺ RNA.

The decapped poly(A)⁺ RNA and 3 nmol of a chimeric DNA-RNA oligonucleotide (5'-dG-dG-dG-dG-dA-dA-dT-dT-dC-dG-dA-G-G-A-3') were dissolved in a solution containing 50 mM Tris-hydrochloric acid buffer (pH 7.5), 0.5 mM ATP, 5 mM MgCl₂, 10 mM 2-mercaptoethanol, and 25% polyethylene glycol, whereto was added 50 units of T4RNA ligase and a total 30 µl volume of the resulting mixture was reacted at 20°C for 12 hours. After the reaction mixture was subjected to phenol extraction followed by ethanol precipitation, the pellet was dissolved in water to obtain a chimeric-oligo-capped poly(A)⁺ RNA.

After digestion of a vector pKA1 (Japanese Patent Kokai Publication No. 1992-117292) developed by the present inventors with KpnI, about 60 dT tails were added using a terminal transferase. A vector primer to be used below was prepared by digestion of this addition product with EcoRV to remove a dT tail at one side.

After 6 µg of the previously-prepared chimeric-oligo-capped poly(A)⁺ RNA was annealed with 1.2 µg of the vector primer, the resulting mixture was dissolved in a solution containing 50 mM Tris-hydrochloric acid buffer (pH 8.3), 75 mM KCl, 3 mM MgCl₂, 10 mM dithiothreitol, and 1.25 mM dNTP (dATP + dCTP + dGTP + dTTP), 200 units of a transcriptase (GIBCO-BRL) were added, and the reaction in a total 20 µl volume was run at 42°C for one hour. After the reaction mixture was subjected to phenol extraction followed by ethanol precipitation, the pellet was dissolved in a solution containing 50 mM Tris-hydrochloric acid buffer (pH 7.5), 100 mM NaCl, 10 mM MgCl₂, and 1 mM dithiothreitol. Thereto were added 100 units of EcoRI and a total 20 µl volume of the resulting mixture was reacted at 37°C for one hour. After the reaction mixture was subjected to phenol extraction followed by ethanol precipitation, the pellet was dissolved in a solution containing 20 mM Tris-hydrochloric acid buffer (pH 7.5), 100 mM KCl, 4 mM MgCl₂, 10 mM (NH₄)₂SO₄, and 50 µg/ml of the bovine serum albumin. Thereto were added 60 units of an *Escherichia coli* DNA ligase and the resulting mixture was reacted at 16°C for 16 hours. To the reaction mixture were added 2 µl of 2 mM dNTP, 4 units of an *Escherichia coli* DNA polymerase I, and 0.1 unit of an *Escherichia coli* DNase H and the resulting mixture was reacted at 12°C for one hour and then at 22°C for one hour.

Next, the cDNA-synthesis reaction solution was used for transformation of an *Escherichia coli* DH12S (GIBCO-BRL). The transformation was carried out by an electroporation method. A portion of the transformant was sprayed on the 2xYT agar culture medium containing 100 µg/ml ampicillin and the mixture was incubated at 37°C overnight. A colony formed on the agar medium was picked up at random and inoculated on 2 ml of the 2xYT culture medium containing 100 µg/ml ampicillin. After incubation at 37°C for 2 hours, the mixture was infected with a helper phage MK13KO7 (Pharmacia) and incubated further at 37°C overnight. The culture solution was centrifuged to separate the mycelia and the supernatant, wherein a double-stranded DNA was isolated from the mycelia by the alkaline hydrolysis method and a single-stranded plasmid DNA from the supernatant according to the conventional method. After double digestion with EcoRI and NotI, the double-stranded plasmid DNA was subjected to 0.8% agarose gel electrophoresis to determine the size of the cDNA insert. On the other hand, after the sequence reaction using an M13 universal primer labeled with a fluorescent dye and a Taq polymerase (a kit of Applied Biosystems), the single-stranded phage DNA was examined with a fluorescent DNA sequencer (Applied Biosystems) to determine the about 400 bp base sequence at the 5'-terminus of the cDNA. The sequence data were filed as the Homo·Protein cDNA Bank database.

### CDNA Cloning

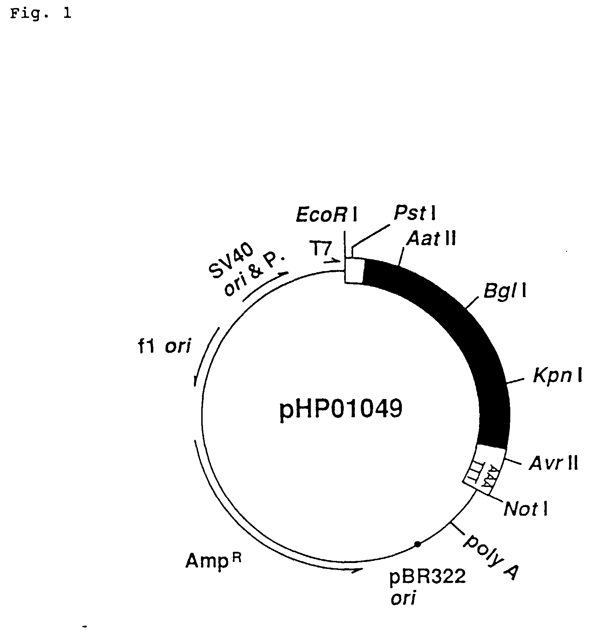

The base sequencing of the clones selected at random from the above-mentioned cDNA library was carried out and the obtained base sequence was converted to three frames of the amino acid sequence, which were subjected to a search of the protein data base. The analysis software used was GENETYX-MAC (Software Development). As the result, a protein encoded by the clone HP01049 was revealed to have the similarity to the rat galectin-4 in the amino acid sequence level. The structure of this plasmid is depicted in Figure 1. The structure consisting of a 56-bp 5'-nontranslation region, a 972-bp open reading frame, an 85-bp 3'-nontranslation region, and a 37-bp poly(A) tail (Sequence No. 3) was found from the determination of the whole base sequence of the cDNA insert. The open reading frame codes for a protein consisting of 323 amino acid residues and the search of the protein data base using this sequence revealed such a high 76.3% similarity to the rat galectin-4 amino acid sequence over the whole regions. Table 1 shows the comparison between the amino acid sequence of the human galectin-4-like protein of the present invention (HS) and that of the rat galectin-4 (RN). Therein, the marks of - (minus), * (asterisk) and. (dot) represent a gap, an amino acid residue identical with the protein of the present invention, and an amino acid residue similar to the protein of the present invention, respectively.

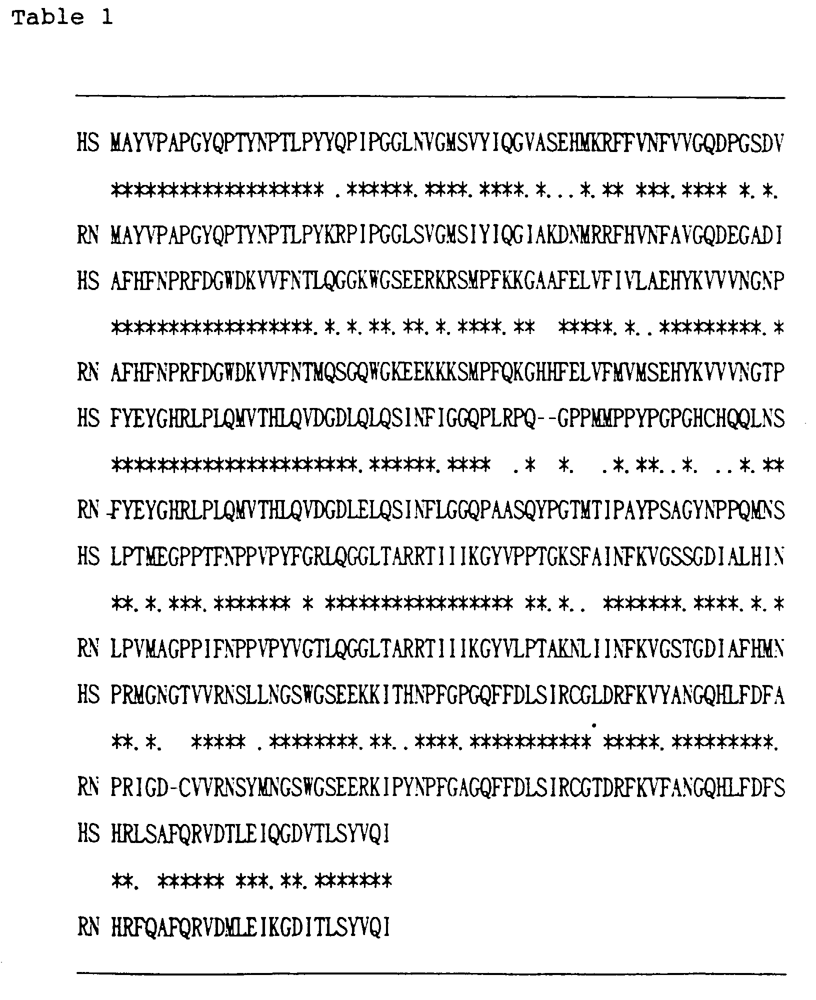

Hereupon, the search of the base sequence databases GenBank™/EMBL/DDBJ using the obtained-cDNA sequence revealed that the EST database has registered a cDNA partial sequence (Accession Mo. D25577) partially consistent with the 3'-nontranslation region (No. 1022 to No. 1113) in the cDNA of the present invention represented by Sequence No. 3. Nevertheless, the consistency of a partial sequence does not assure that said fragment and the full-length cDNA of the present invention originate from the same mRNA. Furthermore, only this sequence does not indicate the amino acid sequence as well as the function of the putatively encoded protein.

### Protein synthesis by In Vitro Translation

The vector pHP01049 having the cDNA of the present invention was used for in vitro translation with a T_{N}T rabbit reticulocyte lysate kit (Promega). In this case, [³⁵S]methionine was added to label the expression product with a radioisotope. Each of the reactions was carried out according to the protocols attached to the kit. Two micrograms of the plasmid pHP01049 was reacted at 30°C for 90 minutes in a total 100 µl volume of the reaction mixture containing 50 µl of the T_{N}T rabbit reticulocyte lysate, 4 µl of a buffer solution (attached to the kit), 2 µl of an amino acid mixture (Met-free), 8 µl of [³⁵S]methionine (Amersham) (0.37 Mbq/µl), 2 µl of T7RNA polymerase, and 80 U of RNasin. To 3 µl of the resulting reaction mixture was added 2 µl of the SDS sampling buffer (125 mM Tris-hydrochloric acid buffer, pH 6.8, 120 mM 2-mercaptoethanol, 2% SDS solution, 0.025% bromophenol blue, and 20% glycerol) and the resulting mixture was heated at 95°C for 3 minutes and then subjected to SDS-polyacrylamide gel electrophoresis. Determination of the molecular weight of the translation product by carrying out the autoradiography indicated that the cDNA of the present invention yielded the translation product with the molecular mass of about 36 kDa. This value is consistent with the molecular weight of 35940 predicted for the putative protein from the base sequence represented by Sequence No. 3, thereby indicating that the cDNA certainly codes for the protein represented by Sequence No. 3.

### Measurement of Lactose-Binding Activity of In-Vitro Translation Product

After 100 ml of a Sepharose-4B gel suspension (Pharmacia) was washed well with 0.5 M sodium carbonate, the gel was suspended in 100 ml of 0.5 M sodium carbonate. Thereto was added 10 ml of a vinyl sulfone and the resulting mixture was gently stirred at room temperature for one hour. After washing with 0.5 M sodium carbonate, the gel was suspended in a solution of 10% lactose and 0.5 M sodium carbonate, and the suspension was stirred gently overnight at room temperature. The resulting gel was washed in order with 0.5 M sodium carbonate, water, and 0.05 M phosphate buffer (pH 7.0). The thus-obtained lactosyl- Sepharose-4B gel was stored at 4°C in the 0.05 M phosphate buffer (pH 7.0) containing 0.02% sodium azide.

By chromatography of 100 µl of the in-vitro translation solution on Sephadex G-75, the unreacted [³⁵S]methionine was removed and the fractions containing the 36-kDa translation product were collected. These fractions were charged in the previously-prepared, lactosyl-Sepharose-4B column (head volume: 4.5 ml), which was washed with 20 ml of a column buffer for the lactose column (20 mM Tris-hydrochloric acid buffer, pH 7.5, 2 mM EDTA, 150 mM NaCl, 4 mM 2-mercaptoethanol, and 0.01% Triton X-100) and eluted with 20 ml of the column buffer containing 0.3 M lactose. As the result, the observation for the eluates to contain the 36-kDa translation product indicated that the protein of the present invention possessed the lactose-binding activity.

### Expression of Fusion Protein by Escherichia coli

After the digestion of 1 µg of the plasmid pHP01049 with 20 units of NotI, blunting was performed by treatment with the Klenow enzyme. Then, after the digestion with PstI, followed by 1% agarose gel electrophoresis, a 1.2-kbp fragment was cut off from the gel.

Next, after 1 µg of pMAL™-c2 (New England Biolabs) was digested with 20 units of HindIII, blunting was performed by treatment with the Klenow enzyme. Then, after the digestion with PstI, followed by 1% agarose gel electrophoresis, a 6.7-kbp DNA fragment was cut off from the gel. The vector fragment and the cDNA fragment were ligated by using a ligation kit and then *Escherichia coli* JM109 was transformed. Plasmid pMALGAL4 was prepared from the transformant and the objective recombinant was identified by the restriction enzyme cleavage map.

A suspension of 10 ml of an overnight-incubated liquid of pMALGAL4/JM109 in 500 ml of the Rich culture medium (contains 10 g of triptone, 5 g of yeast extract, 5 g of NaCl, and 2 g of glucose per one liter) was incubated in a shaker at 37°C and isopropylthiogalactoside was added so as to make 1 mM when A₆₀₀ reached about 0.5. After further incubation at 37°C for 3 hours, the mycelia collected by centrifugation were suspended in 25 ml of a column buffer for amylose column (10 mM Tris-hydrochloric acid, pH 7.4, 200 mM NaCl, and 1 mM EDTA). After sonication, the suspension was centrifuged and the supernatant was charged into an amylose column (New England Biolabs) with a 3.5-ml head volume. After the column was washed with an 8-fold column volume of the column buffer, a maltose-binding protein/galectin-4-like protein fusion protein was eluted with 20 ml of the column buffer containing 10 mM maltose to afford 10.9 mg of the fusion protein. The SDS-polyacrylamide electrophoresis of this fusion protein indicated a single band at the position of about 81 kDa. This molecular mass value is consistent with the molecular weight predicted for the maltose-binding protein/galectin-4-like protein fusion protein.

### Measurement of Lactose-Binding Activity of Fusion Protein

The above-prepared fusion protein was charged in the previously-prepared, lactosyl-Sepharose-4B column (head volume: 4.5 ml), which was washed with 20 ml of a column buffer for the lactose column and eluted with 20 ml of the column buffer containing 0.3 M lactose. The SDS-polyacrylamide electrophoresis of the eluted protein recognized a single band at a 81-kDa position, indicating that the maltose-binding protein/galectin-4 fusion protein obtained by the *Escherichia coli* expression possessed the lactose-binding activity.

### Expression of Galectin-4-Like Protein by Escherichia coli

After the digestion of 1 µg of the plasmid pHP01049 with 20 units of NotI, blunting was performed by treatment with the Klenow enzyme. Then, after the digestion with AatII, followed by 0.8%% agarose gel electrophoresis, an about 1-kbp fragment was cut off from the gel. Next, after 1 µg of the *Escherichia coli* expression vector pMPRA3 (Japanese Patent Kokai Publication No. 1990-182186) having tac promoter, a metapyrocatechase SD sequence, and rrnBT1T2 terminator was digested with 20 units of AatII and with SmaI, followed by 0.8% agarose gel electrophoresis, an about 2.8-kbp DNA fragment was cut off from the gel. Both cDNA fragments were ligated by using a ligation kit and then *Escherichia coli* JM109 was transformed. Plasmid pMAKGAL4-AstII was prepared from the transformant and the objective recombinant was identified by the restriction enzyme cleavage map.

Two strands of an oligonucleotide primer PR1 (5'-GGGACGTCATGGCCTATGTCCCCGCACC-3') and PR2 (5'-GGCGACGTCTGAGCCCGGATCCTGCCC-3') were synthesized using a DNA synthesizer (Applied Biosystems) according to the attached protocol. The 5'-translation region was amplified by the PCR kit (TAKARA SHUZO) using 1 ng of plasmid pHP01049 as well as 100 pmole each of primers PR1 and PR2. After the phenol extraction and ethanol extraction, followed by the digestion with 20 units of AstII (TOYOBO), the reaction product was subjected to 1.5% agarose electrophoresis, cutting off of an about 190-bp DNA fragment, and purification.

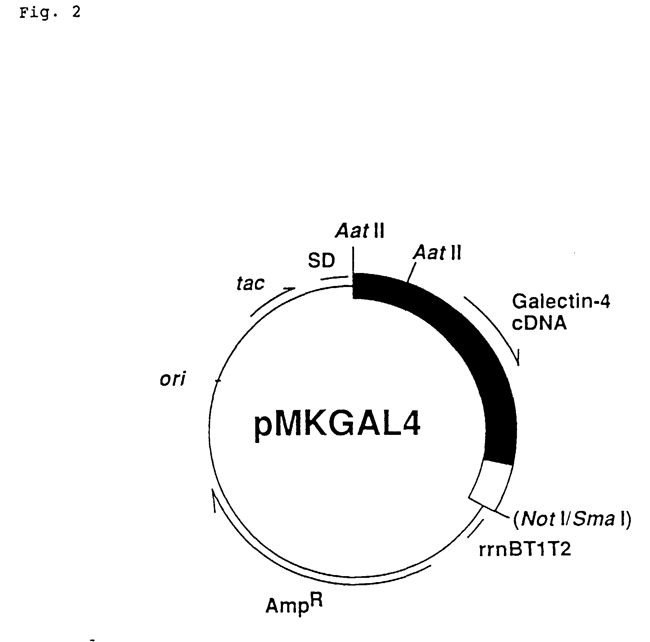

After 1 µg of plasmid pMAKGAL4-AatII was digested with 20 units of AatII, a 3.8-kbp DNA fragment was cut off from the gel. This DNA fragment and the about 190-bp DNA fragment previously prepared by PCR were ligated by using a ligation kit and then *Escherichia coli* JM109 was transformed. Plasmid pMALGAL4 was prepared from the transformant and the objective recombinant was identified by the restriction enzyme cleavage map. Figure 2 depicts the structure of the obtained plasmid.

A suspension of 10 ml of an overnight-incubated liquid of pMALGAL4/JM109 in 100 ml of the LB culture medium containing 100 µg/ml of ampicillin was incubated in a shaker at 37°C and isopropylthiogalactoside was added so as to make 1 mM when A₆₀₀ reached about 0.5. After further incubation at 37°C for 3 hours, the mycelia collected by centrifugation were suspended in 25 ml of the column buffer for lactose column. After sonication, the suspension was centrifuged and the supernatant was charged into the previously prepared, lactosyl-Sepharose-4B column (a 4.5-ml head volume). The column was washed with 20 ml of the column buffer for lactose column and then eluted with 20 ml of the column buffer containing 0.3 M lactose. The SDS-polyacrylamide electrophoresis of this eluted protein indicated a single band at the position of 36 kDa. This molecular mass value is consistent with the molecular weight predicted for the human galectin-4-like protein. That is to say, the human galectin-4-like protein expressed by *Escherichia coli* was indicated to possess the lactose-binding activity.

### Probable Industrial Applicability

The present invention provides a human cDNA encoding a galectin-4-like protein and a protein encoded by this human cDNA. Said recombinant protein can be expressed in a large amount by using the cDNA of the present invention. Said recombinant protein can be used as pharmaceuticals, particularly for the treatment of the digestive tract diseases, or as research reagents, particulary as the reagents for the glycogenic research.

Sequence No.: 1

Sequence length: 323

Sequence type: Amino acid

Sequence kind: Protein

Sequence description

Met Ala Tyr Val Pro Ala Pro Gly Tyr Gln Pro Thr Tyr Asn Pro Thr
1               5                   10                  15
Leu Pro Tyr Tyr Gln Pro Ile Pro Gly Gly Leu Asn Val Gly Met Ser
            20                  25                  30
Val Tyr Ile Gln Gly Val Ala Ser Glu His Met Lys Arg Phe Phe Val
        35                  40                  45
Asn Phe Val Val Gly Gln Asp Pro Gly Ser Asp Val Ala Phe His Phe
    50                  55                  60
Asn Pro Arg Phe Asp Gly Trp Asp Lys Val Val Phe Asn Thr Leu Gln
65                  70                  75                  80

Gly Gly Lys Trp Gly Ser Glu Glu Arg Lys Arg Ser Met Pro Phe Lys
                85                  90                  95
Lys Gly Ala Ala Phe Glu Leu Val Phe Ile Val Leu Ala Glu His Tyr
            100                 105                 110
Lys-Val Val Val Asn Gly Asn Pro Phe Tyr Glu Tyr Gly His Arg Leu
115 120 125
Pro Leu Gln Met Val Thr His Leu Gln Val Asp Gly Asp Leu Gln Leu
130 135 140
Gln Ser Ile Asn Phe Ile Gly Gly Gln Pro Leu Arg Pro Gln Gly Pro
145 150 155 160
Pro Met Met Pro Pro Tyr Pro Gly Pro Gly His Cys His Gln Gln Leu
165 170 175
Asn Ser Leu Pro Thr Met Glu Gly Pro Pro Thr Phe Asn Pro Pro Val
180 185 190
Pro Tyr Phe Gly Arg Leu Gln Gly Gly Leu Thr Ala Arg Arg Thr Ile
195 200 205
Ile Ile Lys Gly Tyr Val Pro Pro Thr Gly Lys Ser Phe Ala Ile Asn
210 215 220
Phe Lys Val Gly Ser Ser Gly Asp Ile Ala Leu His Ile Asn Pro Arg
225 230 235 240
Met Gly Asn Gly Thr Val Val Arg Asn Ser Leu Leu Asn Gly Ser Trp
245 250 255
Gly Ser Glu Glu Lys Lys Ile Thr His Asn Pro Phe Gly Pro Gly Gln
260 265 270
Phe Phe Asp Leu Ser Ile Arg Cys Gly Leu Asp Arg Phe Lys Val Tyr
275 280 285
Ala Asn Gly Gln His Leu Phe Asp Phe Ala His Arg Leu Ser Ala Phe
290 295 300
Gln Arg Val Asp Thr Leu Glu Ile Gln Gly Asp Val Thr Leu Ser Tyr
305 310 315 320
Val Gln Ile

Sequence No.: 2

Sequence length: 969

Sequence type: Nucleic acid

Strandedness: Double

Topology: Linear

Sequence kind: cDNA to mRNA

Origin:

Sequence description

ATGGCCTATG TCCCCGCACC GGGCTACCAG CCCACCTACA ACCCGACGCT GCCTTACTAC 60
CAGCCCATCC CGGGCGGGCT CAACGTGGGA ATGTCTGTTT ACATCCAAGG AGTGGCCAGC 120
GAGCACATGA AGCGGTTCTT CGTGAACTTT GTGGTTGGGC AGGATCCGGG CTCAGACGTC 180
GCCTTCCACT TCAATCCGCG GTTTGACGGC TGGGACAAGG TGGTCTTCAA CACGTTGCAG 240
GGCGGGAAGT GGGGCAGCGA GGAGAGGAAG AGGAGCATGC CCTTCAAAAA GGGTGCCGCC 300
TTTGAGCTGG TCTTCATAGT CCTGGCTGAG CACTACAAGG TGGTGGTAAA TGGAAATCCC 360
TTCTATGAGT ACGGGCACCG GCTTCCCCTA CAGATGGTCA CCCACCTGCA AGTGGATGGG 420
GATCTGCAAC TTCAATCAAT CAACTTCATC GGAGGCCAGC CCCTCCGGCC CCAGGGACCC 480
CCGATGATGC CACCTTACCC TGGTCCCGGA CATTGCCATC AACAGCTGAA CAGCCTGCCC 540
ACCATGGAAG GACCCCCAAC CTTCAACCCG CCTGTGCCAT ATTTCGGGAG GCTGCAAGGA 600
GGGCTCACAG CTCGAAGAAC CATCATCATC AAGGGCTATG TGCCTCCCAC AGGCAAGAGC 660
TTTGCTATCA ACTTCAAGGT GGGCTCCTCA GGGGACATAG CTCTGCACAT TAATCCCCGC 720
ATGGGCAACG GTACCGTGGT CCGGAACAGC CTTCTGAATG GCTCGTGGGG ATCCGAGGAG 780
AAGAAGATCA CCCACAACCC ATTTGGTCCC GGACAGTTCT TTGATCTGTC CATTCGCTGT 840
GGCTTGGATC GCTTCAAGGT TTACGCCAAT GGCCAGCACC TCTTTGACTT TGCCCATCGC 900
CTCTCGGCCT TCCAGAGGGT GGACACATTG GAAATCCAGG GTGATGTCAC CTTGTCCTAT 960
GTCCAGATC 969

Sequence No.: 3

Sequence length: 1113

Sequence type: Nucleic acid

Strandedness: Double

Topology: Linear

Sequence kind: cDNA to mRNA

Origin:

Animal name: Homo sapiens

Cell kind: Stomach cancer tissue

Clone name: HP01049

Sequence characteristics:

Characterization code: CDS

Existence position: 57..1029

Characterization method: E

Sequence description

ATCTCCCACT CCTGCAGCTC TTCTCACAGG ACCAGCCACT AGCGCAGCCT CGAGCG ATG 59
Met
1
GCC TAT GTC CCC GCA CCG GGC TAC CAG CCC ACC TAC AAC CCG ACG CTG 107
Ala Tyr Val Pro Ala Pro Gly Tyr Gln Pro Thr Tyr Asn Pro Thr Leu
            5                   10                  15

CCT TAC TAC CAG CCC ATC CCG GGC GGG CTC AAC GTG GGA ATG TCT GTT 155
Pro Tyr Tyr Gln Pro Ile Pro Gly Gly Leu Asn Val Gly Met Ser Val
        20                  25                  30
TAC ATC CAA GGA GTG GCC AGC GAG CAC ATG AAG CGG TTC TTC GTG AAC 203
Tyr Ile Gln Gly Val Ala Ser Glu His Met Lys Arg Phe Phe Val Asn
    35                  40                  45
TTT GTG GTT GGG CAG GAT CCG GGC TCA GAC GTC GCC TTC CAC TTC AAT 251
Phe Val Val Gly Gln Asp Pro Gly Ser Asp Val Ala Phe His Phe Asn
50                  55                  60                  65
CCG CGG TTT GAC GGC TGG GAC AAG GTG GTC TTC AAC ACG TTG CAG GGC 299
Pro Arg Phe Asp Gly Trp Asp Lys Val Val Phe Asn Thr Leu Gln Gly
                70                  75                  80
GGG AAG TGG GGC AGC GAG GAG AGG AAG AGG AGC ATG CCC TTC AAA AAG 347
Gly Lys Trp Gly Ser Glu Glu Arg Lys Arg Ser Met Pro Phe Lys Lys
            85                  90                  95
GGT GCC GCC TTT GAG CTG GTC TTC ATA GTC CTG GCT GAG CAC TAC AAG 395
Gly-Ala Ala Phe Glu Leu Val Phe Ile Val Leu Ala Glu His Tyr Lys
100 105 110
GTG GTG GTA AAT GGA AAT CCC TTC TAT GAG TAC GGG CAC CGG CTT CCC 443
Val Val Val Asn Gly Asn Pro Phe Tyr Glu Tyr Gly His Arg Leu Pro
115 120 125
CTA CAG ATG GTC ACC CAC CTG CAA GTG GAT GGG GAT CTG CAA CTT CAA 491
Leu Gln Met Val Thr His Leu Gln Val Asp Gly Asp Leu Gln Leu Gln
130 135 140 145
TCA ATC AAC TTC ATC GGA GGC CAG CCC CTC CGG CCC CAG GGA CCC CCG 539
Ser Ile Asn Phe Ile Gly Gly Gln Pro Leu Arg Pro Gln Gly Pro Pro
150 155 160
ATG ATG CCA CCT TAC CCT GGT CCC GGA CAT TGC CAT CAA CAG CTG AAC 587
Met Met Pro Pro Tyr Pro Gly Pro Gly His Cys His Gln Gln Leu Asn
165 170 175
AGC CTG CCC ACC ATG GAA GGA CCC CCA ACC TTC AAC CCG CCT GTG CCA 635
Ser Leu Pro Thr Met Glu Gly Pro Pro Thr Phe Asn Pro Pro Val Pro
180 185 190

TAT TTC GGG AGG CTG CAA GGA GGG CTC ACA GCT CGA AGA ACC ATC ATC 683
Tyr Phe Gly Arg Leu Gln Gly Gly Leu Thr Ala Arg Arg Thr Ile Ile
195 200 205
ATC AAG GGC TAT GTG CCT CCC ACA GGC AAG AGC TTT GCT ATC AAC TTC 731
Ile Lys Gly Tyr Val Pro Pro Thr Gly Lys Ser Phe Ala Ile Asn Phe
210 215 220 225
AAG GTG GGC TCC TCA GGG GAC ATA GCT CTG CAC ATT AAT CCC CGC ATG 779
Lys Val Gly Ser Ser Gly Asp Ile Ala Leu His Ile Asn Pro Arg Met
230 235 240
GGC AAC GGT ACC GTG GTC CGG AAC AGC CTT CTG AAT GGC TCG TGG GGA 827
Gly Asn Gly Thr Val Val Arg Asn Ser Leu Leu Asn Gly Ser Trp Gly
245 250 255
TCC GAG GAG AAG AAG ATC ACC CAC AAC CCA TTT GGT CCC GGA CAG TTC 875
Ser Glu Glu Lys Lys Ile Thr His Asn Pro Phe Gly Pro Gly Gln Phe
260 265 270
TTT GAT CTG TCC ATT CGC TGT GGC TTG GAT CGC TTC AAG GTT TAC GCC 923
Phe Asp Leu Ser Ile Arg Cys Gly Leu Asp Arg Phe Lys Val Tyr Ala
275 280 285
AAT GGC CAG CAC CTC TTT GAC TTT GCC CAT CGC CTC TCG GCC TTC CAG 971
Asn Gly Gln His Leu Phe Asp Phe Ala His Arg Leu Ser Ala Phe Gln
290 295 300 305
AGG GTG GAC ACA TTG GAA ATC CAG GGT GAT GTC ACC TTG TCC TAT GTC 1019
Arg Val Asp Thr Leu Glu Ile Gln Gly Asp Val Thr Leu Ser Tyr Val
310 315 320

CAG ATC TAATCTATTC CTGGGGCCAT AACTCATGGG AAAACAGAAT TATCC 1070
Gln Ile

CCTAGGACTC CTTTCTAAGC CCCTAATAAA ATGTCTGAGG GTG 1113

## Claims

1. A protein comprising an amino acid sequence represented by Sequence No. 1.

2. A cDNA encoding an amino acid sequence represented by Sequence No. 1.

3. A cDNA claimed in Claim 2 comprising a base sequence represented by Sequence No. 2.

4. A cDNA claimed in Claim 3 comprising a base sequence represented by Sequence No. 3.
